# EUROPÄISCHE PATENTSCHRIFT

(11) **EP 2 420 845 B1**
(45) Veröffentlichungstag und Bekanntmachung des Hinweises auf die Patenterteilung: **10.12.2014**
(21) Anmeldenummer: 10008643.8
(22) Anmeldetag: 19.08.2010
(51) Int. Cl.: G01N 35/00, G01N 1/10, G01N 33/28

(54) **System zum Erstellen / Sammeln, Speichern und Zugänglichmachen von Daten einer elektrischen Leistungseinrichtung, welche eine Kühl- oder Arbeitsflüssigkeit enthält**
System for generating / collecting, saving and making available data from an electric facility comprising a cooling or working liquid
Système d'établissement / collecte, de stockage et d'accès aux données d'un dispositif de puissance électrique comprenant un liquide de refroidissement ou de travail

(43) Veröffentlichungstag der Anmeldung: 22.02.2012
(73) Patentinhaber: ABB AG, 68309 Mannheim (DE)
(72) Erfinder: Fantana, Nicolaie, L., 69124 Heidelberg (DE); Kouzmine, Oleg, 40217 Düsseldorf (DE); Werle, Peter, 29664 Walsrode (DE); Radigk, Cornelia, 06124 Halle (DE)

(56) Entgegenhaltungen:
- DE-A1-102008 052 693
- DE-U1-202008 014 618
- US-A- 5 258 310
- US-A- 5 936 715
- US-A1- 2005 005 674
- US-A1- 2009 026 907
- US-B1- 6 199 436
- US-B1- 6 475 443
- US-B1- 6 494 617

## Beschreibung

Die Erfindung betrifft ein System zum Erstellen / Sammeln, Speichern und Zugänglichmachen von Daten einer elektrischen Leistungseinrichtung, welche eine Kühl- oder Arbeitsflüssigkeit enthält.

Elektrische Leistungs-Einrichtungen der Energieerzeugung und industrielle Leistungs-Einrichtungen enthalten vielfach Kühlflüssigkeiten oder Arbeitsflüssigkeiten, wie Öl. Ein typisches Beispiel hierfür ist ein mit Öl gefüllter Leistungstransformator. Um den ordnungsgemäßen Betrieb der elektrischen Leistungs-Einrichtung dauerhaft sicherzustellen, ist es erforderlich, der Kühl- oder Arbeitsflüssigkeit in bestimmten zeitlichen Abständen eine Flüssigkeitsprobe zu entnehmen (Service). Ein mit der entnommenen Flüssigkeitsprobe gefüllter Probeentnahme-Behälter wird danach in ein entferntes Labor transportiert, wo eine ausführliche Diagnose und Analyse der entnommenen Flüssigkeitsprobe erfolgen kann. Als Probeentnahme-Behälter können einerseits Flaschen aus Aluminium, Stahl, Glas oder Kunststoff verwendet werden, andererseits auch Spritzen oder zweiseitig offene Behälter.

Um eine genaue Zuordnung zwischen der elektrischen Leistungs-Einrichtung einerseits und der entnommenen Flüssigkeitsprobe anderseits festzuhalten, ist es üblich, die relevanten Daten, wie Eigentümer und kennzeichnende Daten der elektrischen Leistungs-Einrichtung, Entnahmeort und gewünschte Analysenwerte auf einem am Probeentnahme-Behälter befestigten Aufkleber oder einer Plakette niederzuschreiben. Dabei besteht das Risiko eines Verlustes dieser Plakette oder einer Beschä-digung dieses Aufklebers. Des Weiteren ist es allgemein bekannt, einen Behälter mit einem Behälter-Tag zu versehen, welcher einen Lese-Speicherbereich aufweist, via Funk auslesbar ist und eine eindeutige Identifikation des Behälters ermöglicht.

So ist im Dokument US 6 494 617 B1 ein Zustandsermittlungsgerät unter anderem auch für einen Öltransformator offenbart, wobei anhand der Messung des Fluides beziehungsweise des Öls, welches den Transformator umgibt, Rückschlüsse auf den Betriebszustand des Transformators gezogen werden. Die jeweiligen Sensoren sind hierbei im Einschlußbehälter verteilt.

Im Dokument DE 10 2008 052693 A1 ist ein Transportbehälter für Flüssigkeiten - beispielsweise für medizinische Zwecke - offenbart, welcher zudem eine Dokumentationsvorrichtung beziehungsweise eine Analysevorrichtung für die darin eingefüllte Flüssigkeit umfasst.

Das Patentdokument US 5 936 715 offenbart eine Methode zur optischen Online-Analyse von Öl eines Öltransformafors. Ein Transportbehälter für das Öl wird hierbei vermieden.

Das Patentdokument US 6 475 443 B1 offenbart ein Behältnis für Chemikalien, welches einen intergierten Transponder aufweist. In ähnlicher Weise offenbart auch die Gebrauchsmusterschrift DE 20 2008 014 618 U1 ein Gefäß zur Aufnahme von chemischen Komponenten mit integriertem RFID Transponder.

Der Erfindung liegt die Aufgabe zugrunde, ein optimiertes System zum Erstellen / Sammeln, Speichern und Zugänglichmachen von Daten einer elektrischen Leistungseinrichtung, welche eine Kühl- oder Arbeitsflüssigkeit enthält, anzugeben.

Diese Aufgabe wird erfindungsgemäß gelöst durch ein System zum Erstellen / Sammeln, Speichern und Zugänglichmachen von Daten einer elektrischen Leistungseinrichtung, welche eine Kühl- oder Arbeitsflüssigkeit enthält,
- wobei eine einen Mikroprozessor / Mikrokontroller, einen Speicher, ein Anzeigefeld, ein Eingabefeld und eine Tag-Lese- / Schreib-Vorrichtung aufweisende Erfassungseinrichtung an der elektrischen Leistungseinrichtung befestigt ist,
- wobei ein Probeentnahme-Behälter zur Aufnahme einer der elektrischen Leistungseinrichtung entnommenen Flüssigkeitsprobe vorgesehen ist, an welchem ein Behälter-Tag befestigt ist,
- wobei die Tag-Lese- / Schreib-Vorrichtung nach erfolgter Probeentnahme über eine drahtlose Kommunikationsstrecke
   ∘ die elektrische Leistungseinrichtung kennzeichnende "administrative Daten",
   ∘ die erfolgte Probeentnahme kennzeichnende, nach erfolgter Probeentnahme über das Eingabefeld der Erfassungseinrichtung direkt eingebbare "Diagnose-Daten" an einen Behälter-Tag überträgt.

Die mit der Erfindung erzielbaren Vorteile liegen insbesondere darin, dass zum Erstellen / Sammeln, Speichern und Zugänglichmachen von im Zusammenhang mit einer Probeentnahme stehenden Daten der elektrischen Leistungseinrichtung keine zusätzlichen, von der Service-Person zu transportierenden mobilen Computersysteme erforderlich sind. Die für die Probeentnahme wichtigen, dem Labor mitzu-teilenden Daten stehen vielmehr nach wenigen "Handgriffen" in gebündelter Form zur Verfügung, um sie an den Behälter-Tag des Probeentnahme-Behälters zu transferieren. Es ergeben sich hieraus ein Zeitgewinn und eine Verringerung des Aufwandes bei der Probeentnahme, wodurch insgesamt beachtliche Kostenvorteile hervorgehen. Darüber hinaus ergibt sich eine qualitative Verbesserung beim Service der elektrischen Leistungseinrichtung.

Dabei kann die Erfassungseinrichtung vorteilhaft in ein Steuer- und/oder Überwachungs- und/oder Automatisierungs-System der elektrischen Leistungseinrichtung integriert sein. Die Tag-Lese- / Schreib-Vorrichtung kann bei einer solchen Konfiguration über ein Modul mit diesem Steuer- und/oder Überwachungs- und/oder Automatisierungs-System verbunden sein, wodurch im Zusammenhang mit der Überwachungsfunktionalität des Systems sich ergebende "zusätzliche Leistungseinrichtungs-Daten", wie Betriebswerte betreffend Last und/oder Temperatur und/oder Strom, generierbar und transferierbar sind. Die Tag-Lese- / Schreib-Vorrichtung überträgt dann diese "zusätzliche Leistungseinrichtungs-Daten" nach erfolgter Probeentnahme über die erwähnte drahtlose Kommunikationsstrecke.

In weiterer zweckmäßiger Ausgestaltung des Systems überträgt die Tag-Lese- / Schreib-Vorrichtung nach erfolgter Probeentnahme zusätzlich "historische Daten", welche bei früheren Probeentnahmen ermittelte "Diagnose-Daten" und/oder sich ergebende "zusätzliche Leistungseinrichtungs-Daten" beinhalten.

Generell können die Tag-Lese- / Schreib-Vorrichtung und der Behälter-Tag in Form von RFID-Tags oder NFC-Tags ausgebildet sein.

Eine weitere vorteilhafte Ausbildung des Systems besteht darin, dass an der elektrischen Leistungseinrichtung ein Leistungseinrichtungs-Tag befestigt ist, welcher zumindest die "administrativen Daten" abspeichert und über eine Kommunikationsstrecke mit der Erfassungseinrichtung in Verbindung steht.

Schließlich kann eine drahtlose Kommunikationsstrecke zwischen der Tag-Lese- / Schreib-Vorrichtung und einem Service-Personen-Tag zur Identifizierung der die Probeentnahme durchführenden Service-Person vorliegen.

Weitere zweckmäßige Ausgestaltungen der Erfindung sind in den Unteransprüchen gekennzeichnet.

Die Erfindung wird nachstehend an Hand der in der Zeichnung dargestellten Ausführungsbeispiele erläutert. Es zeigen:
- Fig. 1: eine schematische Skizze zu einer ersten Ausführungsform mit einer eigenständigen Anordnung zum Erstellen / Sammeln, Speichern und Zugänglichmachen von Daten einer elektrischen Leistungseinrichtung,
- Fig. 2: eine beispielhafte Einbaumöglichkeit der Erfassungseinrichtung,
- Fig. 3: eine konkrete Sicht auf ein beispielhaftes Anzeigefeld und Eingabefeld,
- Fig. 4: eine zweite Ausführungsform mit einer beispielhaften Integration der Erfassungseinrichtung in ein Steuer- und/oder Überwachungs- und/oder Automatisierungs-System der elektrischen Leistungseinrichtung.

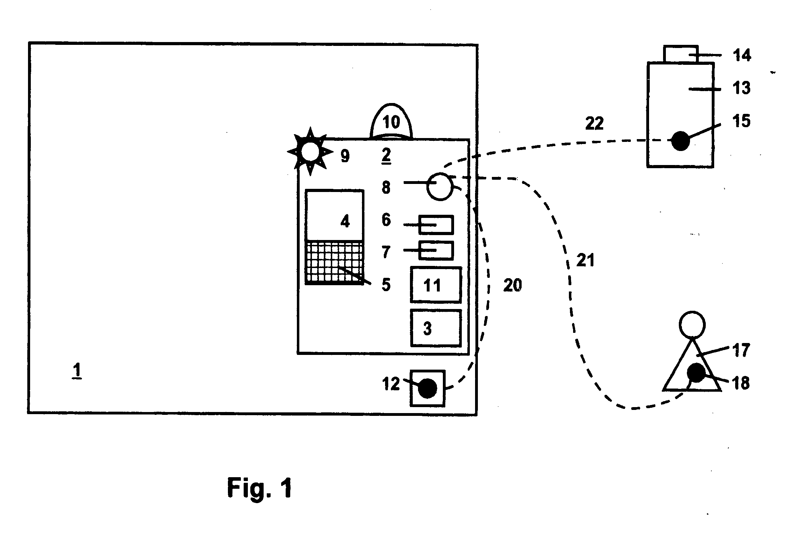

In Fig. 1 ist eine schematische Skizze zu einer ersten Ausführungsform mit einer eigenständigen Anordnung zum Erstellen / Sammeln, Speichern und Zugänglichmachen von Daten einer elektrischen Leistungseinrichtung dargestellt. Es ist eine elektrische Leistungseinrichtung 1 der Energieerzeugung, insbesondere ein Transformator, zu erkennen, welche Öl als Kühl- oder Arbeitsflüssigkeit aufweist. An der elektrischen Leistungseinrichtung 1 sind eine Erfassungseinrichtung 2 und optional ein Leistungseinrichtungs-Tag 12 befestigt. Der Leistungseinrichtungs-Tag 12 kann z. B. unmittelbar am Transformatoren-Kessel unverlierbar befestigt sein oder sich auch innerhalb des Gehäuses der Erfassungseinrichtung 2 befinden.

Die Erfassungseinrichtung 2 enthält die folgenden Baukomponenten:
- einen Mikroprozessor / Mikrokontroller 3
- ein Anzeigefeld 4,
- ein Eingabefeld 5, welches insbesondere in Form eines Tastenfeldes ausgebildet ist,
- eine Bestätigungstaste 6 zur Bestätigung von Daten / Eingaben,
- eine Löschtaste 7 zum Löschen fehlerhafter Daten / Eingaben,
- eine Tag-Lese- / Schreib-Vorrichtung 8 als Standard-Schnittstelle,
- optional eine optische Informations-Signalisierung 9, welche beispielsweise in Form einer LED ausgebildet ist,
- optional eine akustische Informations-Signalisierung 10,
- einen Speicher 11 für die Abspeicherung eingegebener / konfigurierter Daten.

Die aus Anzeigefeld 4, Eingabefeld 5, Bestätigungstaste 6 und Löschtaste 7 bestehende Anordnung kann selbstverständlich auch in Form eines Touch Screens ausgebildet sein.

Der Leistungseinrichtungs-Tag 12 und die Tag-Lese- / Schreib-Vorrichtung 8 sind vorzugsweise als RFID-Tag (Radio Frequency Identification) oder NFC-Tag (Near Field Communication) ausgebildet und mit einem Lese- / Schreib-Speicher versehen. Der Leistungseinrichtungs-Tag 12 kann die Daten der elektrischen Leistungseinrichtung 1, vorzugsweise zusätzlich auch "historische Daten" enthalten und kann insbesondere auch eine eindeutige Identifikation ermöglichen. Mit anderen Worten sind im Leistungseinrichtungs-Tag 12 in Form einer "Lebensakte" die "administrativen Daten" sowie wichtige Ereignisse in der Vergangenheit abgespeichert. Falls der Abstand zwischen Tag-Lese- / Schreib-Vorrichtung 8 und Leistungseinrichtungs-Tag 12 größer ist als dies üblicherweise bei der RFID-Tag-Technologie zulässig ist, ist eine zusätzliche Ausbildung dieser Baueinheiten 8 und 12 in Form eines UHF-Tags oder eines aktiven Tags oder eines drahtlosen (Funk-)Sensor-Knotens realisierbar.

Der Leistungs-Einrichtungs-Tag 12 und die Erfassungseinrichtung 2, hier die Tag-Lese- / Schreib-Vorrichtung 8, sind über eine Kommunikationsstrecke 20 miteinander verbunden, wobei diese Kommunikationsstrecke 20 entweder drahtgebunden oder drahtlos (z. B. in RFID-Form oder in UHF-Form oder in Form aktiver Tags oder in Form drahtloser Funk-Sensor-Knoten) sein kann. Über diese Kommunikationsstrecke 20 können Daten von der Tag-Lese- / Schreibvorrichtung 8 in den Lese- / Schreib-Speicher des Leistungs-Einrichtungs-Tags 12 übertragen werden (z. B. relevante Daten im Zusammenhang mit einer durchgeführten Probeentnahme) und es können umgekehrt Daten des Leistungs-Einrichtungs-Tags 12 zur Tag-Lese- / Schreibvorrichtung 8 übertragen werden (z. B. relevante Daten zur Konfiguration).

Fig. 1 zeigt des Weiteren einen Probeentnahme-Behälter 13 für die Aufnahme einer Kühl- oder Arbeitsflüssigkeit der elektrischen Leistungseinrichtung 1, welcher nach Entnahme der Flüssigkeitsprobe aus der elektrischen Leistungseinrichtung 1 und Befüllung mittels einer Kappe 14 verschließbar ist und einen Behälter-Tag 15 aufweist, welcher vorzugsweise als RFID-Tag (Radio Frequency Identification) oder NFC-Tag (Near Field Communication) ausgebildet und mit einem Lese- / Schreib-Speicher versehen ist. Der Probeentnahme-Behälter 13 wird von einer Service-Person 17 transportiert, wobei die Service-Person 17 optional einen Service-Personen-Tag 18 trägt, vorzugsweise als RFID-Tag oder NFC-Tag ausgebildet, um eine eindeutige Personen-Identifikation derjenigen Person zu ermöglichen, welche eine Probeentnahme der Kühl- oder Arbeitsflüssigkeit der elektrischen Leistungseinrichtung 1 vorgenommen hat.

Dabei ist dem Probeentnahme-Behälter 13 vorzugsweise eine lösbare Sensoreinheit zugeordnet, welche zumindest während der Probeentnahme lösbar am Probeentnahme-Behälter befestigt ist und eine Elektronikgruppe inklusive Prozesseinheit, elektronischer Speicherschaltung und Energieversorgung, eine via Funk zu betreibende Kommunikationsgruppe sowie eine Sensorgruppe aufweist, welche Vorort während der Entnahme der Flüssigkeit vorläufige "ad-hoc-Probe-Sensorik-Daten", wie insbesondere auch die Temperatur der Flüssigkeitsprobe während der Entnahme ermittelt.

Von Interesse sind
- eine optionale (drahtlose) Kommunikationsstrecke 21 zwischen dem Service-Personen-Tag 18 der Service-Person 17 und der Tag-Lese- / SchreibVorrichtung, um eine automatische Identifizierung der die Probeentnahme durchführenden Service-Person zu realisieren 8 sowie
- eine (drahtlose) Kommunikationsstrecke 22 zwischen dem Behälter-Tag 15 und der Tag-Lese- / Schreib-Vorrichtung 8, um den Transfer der für eine Probeentnahme relevanten Daten zum Probeentnahme-Behälter 13 und damit letztlich zum entfernten Labor zu realisieren.

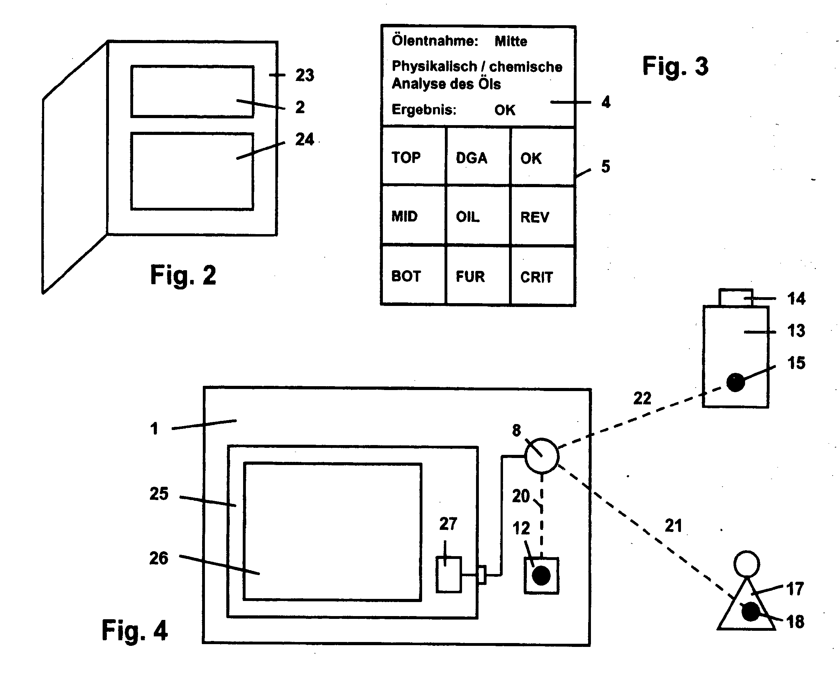

In Fig. 2 ist eine beispielhafte Einbaumöglichkeit der Erfassungseinrichtung dargestellt. Bei diesem Vorschlag weist die elektrische Leistungseinrichtung 1 einen Geräteschrank 23 auf, welcher neben weiteren Leistungseinrichtungs-Geräten 24 unter Anderem auch die Erfassungseinrichtung 2 aufnimmt.

In Fig. 3 ist eine konkrete Sicht auf ein beispielhaftes Anzeigefeld und Eingabefeld dargestellt. Das Eingabefeld 5 weist neun Tasten auf, welche in drei Reihen und drei Spalten angeordnet und in unterschiedlicher Weise beschriftet sind, beispielsweise
- ist die linke obere Taste mit "TOP" beschriftet, was darauf verweist, dass die Flüssigkeitsprobe dem oberen Bereich der elektrischen Leistungseinrichtung 1 entnommen worden ist,
- ist die linke mittlere Taste mit "MID" beschriftet, was darauf verweist, dass die Flüssigkeitsprobe dem mittleren Bereich der elektrischen Leistungseinrichtung 1 entnommen worden ist,
- ist die linke untere Taste ist mit "BOT" beschriftet, was darauf verweist, dass die Flüssigkeitsprobe dem unteren Bereich der elektrischen Leistungseinrichtung 1 entnommen worden ist,
- ist die mittlere obere Taste ist mit "DGA" (Dissolved Gas Analysis) beschriftet, was eine Anforderung an das Labor darstellt, eine Analyse der in der Kühl- oder Arbeitsflüssigkeit gelösten Gase durchzuführen,
- ist die zentrale Taste ist mit "Oil" beschriftet, was an eine Anforderung an das Labor darstellt, eine physikalisch / chemische Analyse des Öls durchzuführen, was z. B. bedeutet, dass u. a. eine Untersuchung der Viskosität, des Säuregehalts, der elektrischen Durchschlagsspannung, der Farbe, der enthaltenen Partikel usw. erfolgen soll,
- ist die mittlere untere Taste ist mit "FÜR" beschriftet, was eine Anforderung an das Labor darstellt, dass die Kühl- oder Arbeitsflüssigkeit auf Furane zu untersuchen ist.
- ist die rechte obere Taste ist mit "OK" beschriftet, was darauf verweist, dass sowohl die elektrische Einrichtung 1 als auch die entnommene Flüssigkeitsprobe nach dem ersten Augenschein von ordnungsgemäßer Beschaffenheit sind,
- ist die rechte mittlere Taste ist mit "REV" beschriftet, was darauf verweist, dass nach dem ersten Augenschein unter Berücksichtigung des Zustandes der elektrischen Einrichtung 1 und/oder der entnommenen Flüssigkeitsprobe eine Revision der elektrischen Einrichtung 1 in naher Zukunft erforderlich ist, d. h. z. B. ein Austausch der Kühl- oder Arbeitsflüssigkeit,
- ist die rechte untere Taste ist mit "CRtT" beschriftet, was darauf verweist, dass nach dem ersten Augenschein unter Berücksichtigung des Zustandes der elektrischen Einrichtung 1 und/oder der entnommenen Flüssigkeitsprobe ein fortgesetzter Betrieb der elektrischen Einrichtung 1 nicht mehr möglich ist, z. B. weil wichtige Teile der elektrischen Einrichtung 1 verrostet sind oder weil Kühl- oder Arbeitsflüssigkeit an einer Stelle der elektrischen Einrichtung austritt.

Im Anzeigefeld 4 werden gewünschte Daten angezeigt, beispielsweise erscheint die Anzeige

| | |
|---|---|
| "Ölentnahme: | Mitte |
| physikalisch / chemische Analyse des Öls | |
| Ergebnis: | OK" |

nachdem die linke mittlere Taste "MD", die zentrale Taste "Oil" und die rechte obere Taste "OK" gedrückt worden sind.

In Fig. 4 ist eine zweite Ausführungsform mit einer beispielhaften Integration der Erfassungseinrichtung in ein Steuer- und/oder Überwachungs- und/oder Automatisierungs-System der elektrischen Leistungseinrichtung dargestellt. Bei diesem Vorschlag besitzt die elektrische Leistungseinrichtung 1 ein Steuer- und/oder Überwachungs- und/oder Automatisierungs-System 25, in welches die Funktionalitäten einiger der vorstehend erläuterten Baukomponenten der Erfassungseinrichtung 2 quasi integriert sind, wie Mikroprozessor / Mikrokontroller 3 und Speicher 11. Das Steuer- und/oder Überwachungs- und/oder Automatisierungs-System 25 weist ein in Form eines Touch Screens ausgebildetes Haupt-Anzeigefeld 26 auf, in welches Anzeigefeld 4, Eingabefeld 5, Bestätigungstaste 6 und Löschtaste 7 integriert sind. Ein Modul 27 des Steuer- und/oder Überwachungs- und/oder Automatisierungs-Systems 25 ist mit der Tag-Lese- / Schreib-Vorrichtung 8 verbunden, wobei die Funktionalität dieses Moduls 27 nachstehend noch erläutert wird.

Zusammenfassend betrachtet sind die Funktionalitäten der unter Fig. 1 erläuterten ersten Ausführungsform mit Erfassungseinrichtung 2 in Form von "Software" in das Steuer- und/oder Überwachungs- und/oder Automatisierungs-System 25 integriert. Bei der unter Figur 4 erläuterten zweiten Ausführungsform mit in das Steuer- und/oder Überwachungs- und/oder Automatisierungs-System 25 integrierter Erfassungseinrichtung 2 können im Vergleich zur ersten Ausführungsform gemäß Fig. 1 "zusätzliche Leistungseinrichtungs-Daten" generiert, d. h. erstellt / gesammelt und zugänglich gemacht werden, welche sich im Zusammenhang mit der Überwachungsfunktionalität des Systems 25 ergeben und über das erwähnte Modul 27 aus dem System 25 "ausgelesen" und in die eigentliche, im System 25 integrierte Erfassungseinrichtung 2 ("softwaremäßig") transferiert werden. Diese "zusätzliche Leistungseinrichtungs-Daten" können in einem späteren Schritt zusätzlich über die drahtlose Kommunikationsstrecke 22 zum Probeentnahme-Behälter 13 übertragen werden, wie insbesondere:
- Betriebswerte, wie Durchschnittswerte für die Last,
- Betriebswerte, wie Durchschnittswerte für die Temperatur,
- Betriebswerte, wie Durchschnittswerte für den Strom,
- Anzahl und Spezifikation der Überlasten,
- Auftreten und Spezifikation von "hot Spots" (Abschnitte der elektrischen Leistungseinrichtung mit übermäßiger Temperatur-Belastung), bei welchen vorgegebene Grenzwerte überschritten worden sind,
- Angabe von Stromwerten, welche vorgegebene Grenzwerte überschritten haben

Nachzutragen ist noch, dass die vorstehenden Erläuterungen zur ersten Ausführungsform gemäß Fig. 1 mit Erfassungseinrichtung 2 im Hinblick auf den Abstand zwischen Tag-Lese- / Schreib-Vorrichtung 8 und Leistungseinrichtungs-Tag 12 in gleicher Art und Weise auch für die zweite Ausführungsform gemäß Fig. 4 gelten. Es ist in gleicher Weise eine zusätzliche Ausbildung dieser Baueinheiten 8 und 12 in Form eines UHF-Tags oder eines aktiven Tags oder eines drahtlosen Sensor-Knotens realisierbar.

Über die Kommunikationsstrecke 20 (entweder drahtgebunden oder drahtlos, z. B. in RFID-Form oder in UHF-Form oder in Form aktiver Tags oder in Form drahtloser Sensor-Knoten) können Daten von der Tag-Lese- / Schreibvorrichtung 8 in den Lese-/ Schreib-Speicher des Leistungs-Einrichtungs-Tags 12 übertragen werden (z. B. relevante Daten im Zusammenhang mit einer durchgeführten Probeentnahme) und es können umgekehrt Daten des Leistungs-Einrichtungs-Tags 12 zur Tag-Lese- / Schreibvorrichtung 8 übertragen werden (z. B. relevante Daten zur Konfiguration)

Es ist erforderlich, das erläuterte System zum Erstellen / Sammeln, Speichern und Zugänglichmachen von Daten einer elektrischen Leistungseinrichtung zu konfigurieren. Während einer Konfigurationsphase der Erfassungseinrichtung 2 respektive des Steuer- und/oder Überwachungs- und/oder Automatisierungs-Systems 25
- wird die Funktionalität des Eingabefeldes 5 respektive des Haupt-Anzeigefeldes 26 festgelegt und dauerhaft abgespeichert (im Speicher 11), d. h. es wird im Einzelnen festgelegt welche Taste mit welcher Beschriftung belegt wird,
- wird die Funktionalität des Anzeigefeldes 4 respektive Haupt-Anzeigefeldes 26 festgelegt und dauerhaft abgespeichert, d. h. es werden anzuzeigende Texte respektive anzuzeigende Symbolik festgelegt,
- werden die "administrativen Daten" der elektrischen Leistungseinrichtung 1 eingelesen, wie
   o eindeutige Identifikation der elektrischen Leistungseinrichtung 1,
   o Aufstellungsort (z. B. in Form von GPS-Daten) und Eigentümer der elektrischen Leistungseinrichtung 1,
   o kennzeichnende Daten der elektrischen Leistungseinrichtung 1, wie Seriennummer, Leistung, Spannung, usw.
   o Flüssigkeitstyp der Kühl- oder Arbeitsflüssigkeit, z. B. Öltyp.

Die Konfiguration kann dabei unter Zuhilfenahme des Leistungseinrichtungs-Tags 12 erfolgen, welcher die "administrativen Daten" enthält. Eine für die Konfiguration erforderliche Konfigurations-Datei kann dabei z. B. über eine Standard-Schnittstelle der Erfassungseinrichtung 2 respektive des Steuer- und/oder Überwachungs- und/oder Automatisierungs-Systems 25, z. B. in Form einer USB-Schnittstelle ausgebildet, zur Verfügung gestellt werden. Eine weitere Möglichkeit besteht darin, dass die Service-Person 17 einen die für die Konfiguration erforderliche Konfigurations-Datei enthaltenden Initialisierungs-Tag (z. B. RFID-Tag) der Tag-Lese- / Schreib-Vorrichtung 8 annähert, worauf ein Einlesen der Konfigurations-Datei erfolgt. Das hat den Vorteil, dass keine zusätzliche Schnittstelle erforderlich ist. Dieser Initialisierungs-Tag wird z. B. von einem Service-Ingenieur vorbereitet / erstellt.

Nachfolgend wird die Arbeitsweise des Systems zum Erstellen / Sammeln, Speichern und Zugänglichmachen von Daten einer elektrischen Leistungseinrichtung näher beschrieben: ,

### Schritt A:

Die Service-Person 17 führt eine Probeentnahme an der elektrischen Leistungseinrichtung 1 durch und der Probeentnahme-Behälter 13 ist mit der Probe der Kühl- oder Arbeitsflüssigkeit gefüllt.

### Schritt B:

Die Service-Person 17 gibt mithilfe des Eingabefeldes 5 / Anzeigefeldes 4 respektive des Haupt-Anzeigefeldes 26 folgende "Diagnose-Daten" in die Erfassungseinrichtung 2 respektive das Steuer- und/oder Überwachungs- und/oder Automatisierungs-System 25 ein:
- Entnahmeort der Probeentnahme an der elektrischen Leistungseinrichtung 1, z. B. "im Bodenbereich", "im oberen Bereich", "im mittleren Bereich", "Entnahmehahn xxx",
- Datum und Uhrzeit der Probeentnahme,
- Entnahmemenge,
- Name der Service-Person 17, welche die Flüssigkeitsprobe entnommen hat,
- optional einen während der Entnahme erzeugten eindeutigen Bestätigungscode,
- wichtige (insbesondere kritische) Beobachtungen respektive Kommentare der Service-Person 17 während der Entnahme der Flüssigkeitsprobe,
- grundlegende, auf den ersten Blick von der Service-Person 17 erkennbare Beobachtungen, wie "Öl-Zustand: OK", "ÖI-Zustand: Revision erforderlich" oder Öt-Zustand: kritisch", wie oben erläutert,
- die vom entfernten Labor zu ermittelnden, gewünschten Analysewerte, z. B. in der Flüssigkeit gelöste Gase (z. B. gelöster Wasserstoff), durchzuführende chemische Tests, Furane, usw., wie unter Anderem auch
   o enthaltenes Azetylen,
   o enthaltenes Ethin,
   o enthaltenes Wasser,
   o enthaltenes Kohlenmonoxyd,
   o enthaltenes Kohlendioxyd,
   o enthaltener Sauerstoff,
   o enthaltener Stickstoff,
   o ermittelte Leitfähigkeit,
   o ermittelter Säuregehalt.

Im einfachsten Fall drückt die Service-Person lediglich eine Taste der linken Reihe, eine Taste der mittleren Reihe und eine Taste der rechten Reihe, wie vorstehend unter Fig. 4 erläutert.

Die Eingabe des Namens der Service-Person 17, welche die Flüssigkeitsprobe entnommen hat, kann alternativ und vorteilhaft auch selbsttätig und direkt über die Kommunikationsstrecke 21 zwischen Service-Personen-Tag 18 und der Tag-Lese- / Schreib-Vorrichtung 8 erfolgen.

### Schritt C:

Die Service-Person 17 bringt den Probeentnahme-Behälter 13 in die Nähe der Tag-Lese- / Schreib-Vorrichtung. Über die drahtlose Kommunikationsstrecke 22 werden von der Tag-Lese- / Schreib-Vorrichtung 8 selbsttätig folgende Daten an den Behälter-Tag 15 des Probeentnahme-Behälters 13 übermittelt:
- die vorstehend im Einzelnen angegebenen "administrativen Daten",
- die vorstehend im Einzelnen angegebenen "Diagnose-Daten",
- die vorstehend im Einzelnen angegebenen "zusätzlichen Leistungseinrichtungs-Daten",
- optional die in Vergangenheit (bei früheren Probeentnahmen) ermittelten "Diagnose-Daten" und/oder "zusätzlichen Leistungseinrichtungs-Daten", auch als "historische Daten" bezeichnet.

Die optische Informations-Signalisierung 9 und/oder die akustische Informations-Signalisierung 10 leisten Hilfestellung bei den vorstehend erläuterten Schritten, d. h. es werden der Service-Person 17 Signale "Bin bereit", "Bin fertig", "Fehler" usw. in optischer und/oder akustischer Art und Weise zur Erleichterung der Menüführung mitgeteilt.

In einem Schritt D kann nun der Transport des Probeentnahme-Behälters zum Labor erfolgen, wo die detaillierte Diagnose und Analyse der entnommenen Flüssigkeitsprobe entsprechend den im Behälter-Tag 15 abgespeicherten Informationen erfolgt.

Wie vorstehend bereits angedeutet, kann die Tag-Lese- / Schreib-Vorrichtung 8 nach erfolgter Probeentnahme in einem Schritt E relevante Daten an den Leistungseinrichtungs-Tag 12 übermitteln (Update), wie z. B. das Datum der Probeentnahme und Kommentare betreffend kritische Größen. Diese aktualisierten Daten stehen dann bei der nächsten Probeentnahme wieder zur Verfügung.

Dabei ist es von Bedeutung, dass die im Speicher 11 der Erfassungseinrichtung 2 abgespeicherten, in der Vergangenheit ermittelten "historische Daten" mit den aktuellen "Diagnose-Daten" und/oder "zusätzliche Leistungseinrichtungs-Daten" verglichen und in Beziehung miteinander gebracht werden können. Durch sinnvolle Verknüpfung der individuellen Daten der entnommenen Flüssigkeitsproben untereinander wird ein den aktuellen Zustand der elektrischen Einrichtung 1 repräsentierendes Gesamtbild erhalten und es können abgeleitet hiervon Maßnahmen vorgeschlagen werden, welche den sicheren Betrieb der elektrischen Einrichtung 1 auch zukünftig ermöglichen. Es lassen sich Trends rechtzeitig erkennen, wobei Risiko-Abschätzungen, Fuzzylogik, "Neural Networks" usw. Verwendung finden können. Es ist eine Visualisierung über das Anzeigefeld 4 respektive Haupt-Anzeigefeld 26 möglich, wie vorstehend erläutert.

Ergänzend zu den vorstehenden Ausführungen ist nachzutragen, dass die Erfassungseinrichtung 2 respektive das Steuer- und/oder Überwachungs- und/oder Automatisierungs-System 25 zusätzlich eine Sprachausgabe für die Ausgabe von Informationen für den Anwender aufweisen kann. Des Weiteren kann ein Spracheingang alternativ oder zusätzlich zum Eingabefeld 5 / Anzeigefeld 4 / Haupt-Anzeigefeld 26 vorgesehen sein, um dem Anwender die Möglichkeit zu geben, Spracheingaben vorzunehmen, d. h. die unter Fig. 4 erläuterten Eingaben können in einem solchen Fall in Form von Spracheingaben erfolgen.

### Bezuoszeichenliste

- 1: Elektrische Leistungseinrichtung, z. B. Transformator
- 2: Erfassungseinrichtung
- 3: Mikroprozessor / Mikrokontroller
- 4: Anzeigefeld
- 5: Eingabefeld
- 6: Bestätigungstaste
- 7: Löschtaste
- 8: Tag-Lese- / Schreib-Vorrichtung
- 9: optische Informations-Signalisierung
- 10: akustische Informations-Signalisierung
- 11: Speicher
- 12: Leistungseinrichtungs-Tag
- 13: Probeentnahme-Behälter für eine Kühl- oder Arbeitsflüssigkeit
- 14: Kappe
- 15: Behälter-Tag
- 16: -
- 17: Service-Person
- 18: Service-Personen-Tag
- 19: -
- 20: Kommunikationsstrecke zwischen Leistungseinrichtungs-Tag 12 und Erfassungseinrichtung 2 (drahtgebunden oder drahtlos)
- 21: Kommunikationsstrecke zwischen Service-Personen-Tag und Tag-Lese- / Schreib-Vorrichtung (drahtlos)
- 22: Kommunikationsstrecke zwischen Tag-Lese- / Schreib-Vorrichtung und Behälter-Tag (drahtlos)
- 23: Geräteschrank
- 24: Leistungseinrichtungs-Geräte
- 25: Steuer- und/oder Überwachungs- / Automatisierungs-System
- 26: Haupt-Anzeigefeld
- 27: Modul

## Patentansprüche

1. System zum Erstellen / Sammeln, Speichern und Zugänglichmachen von Daten einer elektrischen Leistungseinrichtung (1), welche eine Kühl- oder Arbeitsflüssigkeit enthält,
• wobei eine einen Mikroprozessor / Mikrokontroller (3), einen Speicher (11), ein Anzeigefeld (4, 26), ein Eingabefeld (5) und eine Tag-Lese- / Schreib-Vorrichtung (8) aufweisende Erfassungseinrichtung (2, 25) an der elektrischen Leistungseinrichtung (1) befestigt ist,
• wobei ein Probeentnahme-Behälter (13) zur Aufnahme einer der elektrischen Leistungseinrichtung (1) entnommenen Flüssigkeitsprobe vorgesehen ist, an welchem ein Behälter-Tag (15) befestigt ist,
• wobei die Tag-Lese- / Schreib-Vorrichtung (8) dafür geeignet ist, nach erfolgter Probeentnahme über eine drahtlose Kommunikationsstrecke (22)
• die elektrische Leistungseinrichtung (1) kennzeichnende "administrative Daten",
• die erfolgte Probeentnahme kennzeichnende, nach erfolgter Probe-entnahme über das Eingabefeld (5) der Erfassungseinrichtung (2, 25) direkt eingebbare "Diagnose-Daten" an einen Behälter-Tag (16) zu übertragen.
• wobei die Erfassungseinrichtung (2) in ein Steuer- und/oder Überwachungs- und/oder Automatisierungs-System (25) der elektrischen Leistungseinrichtung (1) integriert ist,
• wobei die Tag-Lese- / Schreib-Vorrichtung (8) über ein Modul (27) mit dem Steuer- und/oder Überwachungs- und/oder Automatisierungs-System (25) verbunden ist, wodurch im Zusammenhang mit der Überwachungsfunktionalität des Systems (25) sich ergebende "zusätzliche Leistungseinrichtungs-Daten", wie Betriebswerte betreffend Last und / oder Temperatur und/oder Strom, generierbar und transferierbar sind,
**dadurch gekennzeichnet,**
**dass** die Tag-Lese- / Schreib-Vorrichtung (8) dafür vorgesehen ist, nach erfolgter Probeentnahme über die drahtlose Kommunikationsstrecke (22) zusätzlich "historische Daten" zu übertragen, welche bei früheren Probeentnahmen ermittelte "Diagnose-Daten" und sich ergebende "zusätzliche Leistungseinrichtungs-Daten" beinhalten.

2. System nach Anspruch 1, **dadurch gekennzeichnet, dass** die Tag-Lese- / Schreib-Vorrichtung (8) und der Behälter-Tag (15) in Form von RFID-Tags ausgebildet sind.

3. System nach Anspruch 1, **dadurch gekennzeichnet, dass** die Tag-Lese- / Schreib-Vorrichtung (8) und der Behälter-Tag (15) in Form von NFC-Tags ausgebildet sind.

4. System nach einem der vorstehenden Ansprüche, **dadurch gekennzeichnet, dass** die Erfassungseinrichtung (29) in einem Geräteschrank (23) installiert ist.

5. System nach einem der vorstehenden Ansprüche, **dadurch gekennzeichnet, dass** an der elektrischen Leistungseinrichtung (1) ein Leistungseinrichtungs-Tag (12) befestigt ist, welcher In Form einer "Lebensakte" die "administrativen Daten" sowie wichtige Ereignisse in Vergangenheit abspeichert und über eine Kommunikationsstrecke (20) mit der Erfassungseinrichtung (2) in Verbindung steht.

6. System nach einem der vorstehenden Ansprüche, **gekennzeichnet durch** eine drahtlose Kommunikationsstrecke (21) zwischen der Tag-Lese- / Schreib-Vorrichtung (8) und einem Service-Personen-Tag (18) zur Identifizierung der die Probeentnahme durchführenden Service-Person (17).

## Claims

1. System for creating/collecting, storing and providing access to data from an electric power device (1) which contains a cooling or operating liquid,
• wherein a sensing device (2, 25) which has a microprocessor/microcontroller (3), a memory (11), a display panel (4, 26), an input panel (5) and a tag read/write apparatus (8) is mounted on the electric power device (1),
• wherein a sampling container (13) for holding a liquid sample taken from the electric power device (1) is provided, on which a container tag (15) is mounted,
• wherein the tag read/write apparatus (8) is suitable, when a sample has been taken, for using a wireless communication link (22) to transmit
• "administrative data" denoting the electric power device (1),
• "diagnosis data", denoting the sample taken, which can be input directly using the input panel (5) of the sensing device (2, 25) when a sample has been taken, to a container tag (16),
• wherein the sensing device (2) is integrated in a control and/or monitoring and/or automation system (25) of the electric power device (1),
• wherein the tag read/write apparatus (8) is connected to the control and/or monitoring and/or automation system (25) via a module (27), as a result of which it is possible to generate and transfer "additional power device data" arising in connection with the monitoring functionality of the system (25), such as operational values relating to load and/or temperature and/or current,
**characterized**
**in that** the tag read/write apparatus (8) provided for the purpose of additionally transmitting "historical data", which contain "diagnosis data" ascertained for earlier samples taken and resultant "additional power device data", using the wireless communication link (22) when a sample has been taken.

2. System according to Claim 1, **characterized in that** the tag read/write apparatus (8) and the container tag (15) are in the form of RFID tags.

3. System according to Claim 1, **characterized in that** the tag read/write apparatus (8) and the container tag (15) are in the form of NFC tags.

4. System according to one of the preceding claims, **characterized in that** the sensing device (29) is installed in an equipment cabinet (23).

5. System according to one of the preceding claims, **characterized in that** the electric power device (1) has a power device tag (12) mounted on it which stores the "administrative data" and also important events in the past in the form of a "record file" and is in contact with the sensing device (2) via a communication link (20).

6. System according to one of the preceding claims, **characterized by** a wireless communication link (21) between the tag read/write apparatus (8) and a servicing person tag (18) for identifying the servicing person (17) taking the sample.

## Revendications

1. Système pour créer/collecter, mettre en mémoire et rendre accessibles des données d'un dispositif de puissance électrique (1), qui contient un liquide de refroidissement ou de travail,
* un dispositif d'acquisition (2, 25) présentant un microprocesseur/microcontrôleur (3), une mémoire (11), un volet d'affichage (4, 26), un volet de saisie (5) et un équipement de lecture/écriture de balise (8) étant fixé au dispositif de puissance électrique (1),
* un récipient de prélèvement d'échantillon (13) destiné à accueillir un échantillon de liquide prélevé du dispositif de puissance électrique (1) étant prévu, une balise de récipient (15) étant fixée à celui-ci,
* l'équipement de lecture/écriture de balise (8) étant conçu pour, après qu'un prélèvement d'échantillon ait été effectué, transmettre à une balise de récipient (16), par le biais d'une section de communication (22) sans fil,
* des « données administratives » qui identifient le dispositif de puissance électrique (1),
* des « données de diagnostic » qui identifient le prélèvement d'échantillon effectué, pouvant être saisies directement par le biais du volet de saisie (5) du dispositif d'acquisition (2, 25) après un prélèvement d'échantillon effectué,
* le dispositif d'acquisition (2) étant intégré dans un système de commande et/ou de surveillance et/ou d'automatisation (25) du dispositif de puissance électrique (1),
* l'équipement de lecture/écriture de balise (8) étant relié par le biais d'un module (27) avec le système de commande et/ou de surveillance et/ou d'automatisation (25), les « données de dispositif de puissance supplémentaires » qui résultent en association avec la fonctionnalité de surveillance du système (25), telles que des valeurs opérationnelles concernant la charge et/ou la température et/ou le courant, pouvant ainsi être générées et être transférées,
**caractérisé en ce que** l'équipement de lecture/écriture de balise (8) est prévu pour, après qu'un prélèvement d'échantillon ait été effectué, transmettre en outre des « données historiques » par le biais de la section de communication (22) sans fil, lesquelles contiennent les « données de diagnostic » déterminées lors des prélèvements d'échantillon antérieurs et les « données de dispositif de puissance supplémentaires » qui en résultent.

2. Système selon la revendication 1, **caractérisé en ce que** l'équipement de lecture/écriture de balise (8) et la balise de récipient (15) sont réalisés sous la forme de balises RFID.

3. Système selon la revendication 1, **caractérisé en ce que** l'équipement de lecture/écriture de balise (8) et la balise de récipient (15) sont réalisés sous la forme de balises NFC.

4. Système selon l'une des revendications précédentes, **caractérisé en ce que** le dispositif d'acquisition (29) est installé dans une armoire d'appareil (23).

5. Système selon l'une des revendications précédentes, **caractérisé en ce qu'**une balise de dispositif de puissance électrique (12) est fixée au dispositif de puissance électrique (1), laquelle enregistre les « données administratives » ainsi que les événements importants du passé sous la forme d'un « événement d'activité » et se trouve en liaison avec le dispositif d'acquisition (2) par le biais d'une section de communication (20).

6. Système selon l'une des revendications précédentes, **caractérisé par** une section de communication (21) sans fil entre l'équipement de lecture/écriture de balise (8) et une balise de personne de service (18) destinée à identifier la personne de service (17) qui effectue le prélèvement d'échantillon.
